# EUROPÄISCHE PATENTANMELDUNG

(11) **EP 1 535 602 A1**
(43) Veröffentlichungstag der Anmeldung: **01.06.2005**
(21) Anmeldenummer: 04105138.4
(22) Anmeldetag: 19.10.2004
(51) Int. Cl.: A61K 7/15, A61K 7/32, A61K 7/48

(54) **Kosmetische Formulierungen auf Fettsäurebasis**

(30) Priorität: 26.11.2003 DE 10355712
(71) Anmelder: Beiersdorf AG, 20253 Hamburg (DE)
(72) Erfinder: Traupe, Bernd, 22457, Hamburg (DE); Moeller, Martina, 24558, Henstedt-Ulzburg (DE); Hallmann, Martina, 22417, Hamburg (DE); Jung, Susanne, 22529, Hamburg (DE)

(57) **Zusammenfassung**

Kosmetische oder dermatologische Zubereitungen enthaltend ein oder mehrere 4-Methyl-4-aryl-2-pentanole, dadurch gekennzeichnet, daß sie mit organischen oder anorganischen Basen neutralisierte, geradkettige Fettsäuren mit 14 bis 18 Kohlenstoffatomen enthalten, wobei der pH-Wert der Zubereitungen zwischen 6 und 11 liegt.

## Beschreibung

Die vorliegende Erfindung betrifft kosmetische oder dermatologische Zubereitungen, welche 4-Methyl-4-aryl-2-pentanole enthalten, insbesondere betrifft sie desodorierende Stiftformulierungen, Rasierschäume und -gele sowie Zubereitungen, welche nach einer Rasur auf die Haut aufgetragen werden (sog. After shave Formulierungen), solche Wirkstoffe enthaltend.

4-Methyl-4-aryl-2-pentanole zeichnen sich durch die folgende Strukturformel aus 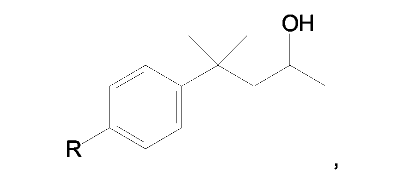 worin R gewählt wird aus der Gruppe -H, -OH, Alkoxygruppen mit 1 bis 10 Kohlenstoffatomen, verzweigte oder unverzweigte, gesättigte oder ungesättigte Alkylreste mit 1 bis 10 Kohlenstoffatomen.

Die antimikrobielle Wirksamkeit von 4-Methyl-4-aryl-2-pentanolen ist an sich bekannt (siehe auch DE 101 43 434). Sie wurde bei in-vitro-Experimenten gefunden, bei denen der Einfluss der reinen Wirkstoffe auf bestimmte Testkeime untersucht wurde. Ferner beschreibt die DE 101 43 434 u. a. auch die Verwendung von 4-Methyl-4-aryl-2-pentanolen zur kosmetischen Behandlung von Körpergeruch verursachenden Mikroorganismen, allerdings ohne weitere Hinweise zu geben, wie die Wirkstoffe in Kosmetika eingearbeitet werden könnten o. ä.

Bei dem Versuch, 4-Methyl-4-aryl-2-pentanole in kosmetische Zubereitungen - wie beispielsweise klassische Aerosolzubereitungen - einzuarbeiten, hat sich allerdings herausgestellt, dass die gefundene Wirksamkeit deutlich hinter der nach den in-vitro-Experimenten erwarteten zurückbleibt, da diese Substanzen aus klassischen Aerosolzubereitungen sehr schnell freigesetzt werden, verdunsten oder in die Haut penetrieren. Durch die schnelle Verdunstung oder Penetration wird der Wirkstoff seiner Wirkstätte entzogen (er geht sozusagen unmittelbar verloren), so dass sich keinerlei schweiß- oder geruchshemmende Wirkung feststellen lässt. Dies zeigt sich z. B. im sog. "Sniff-Test", bei dem per Geruchsbestimmung untersucht wird, ob Zubereitungen ausreichend gegen Schweißgeruch wirken bzw. ob auch nach einer bestimmten Testzeit (beispielsweise nach 24 Stunden) noch kein Schweißgeruch festzustellen ist, also die Wirkung der Deos immer noch anhält.

Aufgabe der vorliegenden Erfindung war es daher, auf einfache und preiswerte Weise zu kosmetischen oder dermatologischen Zubereitungen zu gelangen, welche 4-Methyl-4-aryl-2-pentanole enthalten und sich durch eine gute Deo-Wirksamkeit auszeichnen.

Überraschend und für den Fachmann nicht vorauszusehen war, dass
kosmetische oder dermatologische Zubereitungen enthaltend ein oder mehrere 4-Methyl-4-aryl-2-pentanole, dadurch gekennzeichnet, dass sie eine oder mehrere mit organischen oder anorganischen Basen neutralisierte, geradkettige Fettsäuren mit 14 bis 18 Kohlenstoffatomen enthalten, wobei der pH-Wert der Zubereitungen zwischen 6 und 11 liegt,
den Nachteilen des Standes der Technik abhelfen würden.

Die erfindungsgemäßen Zubereitungen zeichnen sich erstaunlicherweise dadurch aus, daß die 4-Methyl-4-aryl-2-pentanole sehr langsam freigesetzt werden, wobei nur der freigesetzte Anteil verdunsten oder in die Haut penetrieren kann. Dementsprechend zeigen sie eine sehr gute Wirksamkeit gegen Corynebacterium xerosis, Corynebacterium jeikeium, Staphylococus epidermis und Propionibacterium acnes aus und eignen sich hervorragend als kosmetische oder dermatologische Desodorantien.

Gegenstand der Erfindung ist daher ferner auch die
Verwendung von kosmetischen oder dermatologischen Zubereitungen enthaltend eine oder mehrere mit organischen oder anorganischen Basen neutralisierte, geradkettige Fettsäuren mit 14 bis 18 Kohlenstoffatomen zur Verhinderung oder Verzögerung der Penetration von 4-Methyl-4-aryl-2-pentanolen in die Haut.

Erfindungsgemäß besonders vorteilhaftes 4-Methyl-4-aryl-2-pentanol ist das 4-Methyl-4-phenyl-2-pentanol.

Erfindungsgemäß bevorzugt werden die 4-Methyl-4-aryl-2-pentanole (eine oder mehrere Verbindungen) in einem Gehalt von 0,005 bis 10 Gew.-%, insbesondere 0,01 bis 5 Gew.-%, ganz besonders vorteilhaft 0,1 bis 3,0 Gew.-%, jeweils bezogen auf das Gesamtgewicht der Zubereitung, eingesetzt.

Es ist auch von Vorteil, anstatt reiner erfindungsgemäßer 4-Methyl-4-aryl-2-pentanole Mischungen zu verwenden, welche sich durch einen Gehalt an erfindungsgemäßen 4-Methyl-4-aryl-2-pentanolen auszeichen.

Erfindungsgemäß besonders vorteilhafte geradkettige Fettsäuren sind Stearinsäure, Palmitinsäure und/oder Myristinsäure.

Erfindungsgemäß bevorzugt werden die geradkettigen Fettsäuren (eine oder mehrere Verbindungen) in einem Gehalt von 0,005 bis 20 Gew.-%, insbesondere 0,1 bis 10 Gew.-%, jeweils bezogen auf das Gesamtgewicht der Zubereitung, eingesetzt.

Vorteilhafte erfindungsgemäße Zubereitungen sind z. B. Stiftformulierungen, insbesondere Deo-Stiftformulierungen. In Stiftformulierungen im Sinne der vorliegenden Erfindung wird die erfindungsgemäße geradkettige Fettsäure (eine oder mehrere Verbindungen) vorzugsweise mit anorganischen Basen (beispielsweise Natronlauge) neutralisiert, wobei der Verseifungsgrad vorteilhaft aus dem Bereich von 70 bis 100 %, besonders vorteilhaft aus dem Bereich von 75 bis 90 % gewählt wird. Der pH-Wert solcher Zubereitungen liegt vorzugsweise im Bereich von 8,5 bis 11 und ist besonders bevorzugt ca. 10.

Ferner vorteilhaft im Sinne der vorliegenden Erfindung sind schaumförmige bzw. nachschäumende Aerosolformulierungen auf Basis erfindungsgemäßer Fettsäuren, in denen die Fettsäure (eine oder mehrere Verbindungen) vorzugsweise mit organischen Basen (bevorzugt Triethanolamin) neutralisiert ist, wobei der Verseifungsgrad vorteilhaft aus dem Bereich von 100 bis 140 %, besonders vorteilhaft aus dem Bereich von 115 bis 130 % gewählt wird. Der pH-Wert solcher Zubereitungen liegt vorzugsweise im Bereich von 7 bis 10 und ist besonders bevorzugt ca. 8 bis 9.

Unter "schaumförmig" bzw. "nachschäumend" ist im Sinne der vorliegenden Erfindung zu verstehen, dass Gasbläschen (beliebig) verteilt in einer (oder mehreren) flüssigen Phase(n) vorliegen, wobei die Zubereitungen makroskopisch nicht notwendigerweise das Aussehen eines Schaumes haben müssen. Die kosmetischen oder dermatologischen Zubereitungen (im folgenden der Einfachheit halber auch als Schäume bezeichnet) können z. B. makroskopisch sichtbar dispergierte Systeme aus in Flüssigkeiten dispergierten Gasen darstellen. Der Schaumcharakter kann aber beispielsweise auch erst unter einem (Licht-) Mikroskop sichtbar werden. Darüber hinaus sind erfindungsgemäße Schäume - insbesondere dann, wenn die Gasbläschen zu klein sind, um unter einem Lichtmikroskop erkannt zu werden - auch an der starken Volumenzunahme des Systems erkennbar.

Nachschäumende kosmetische Zubereitungen werden zunächst in fließförmiger Form aus einem Aerosolbehälter auf die Haut aufgetragen und entwickeln dort erst nach kurzer Verzögerung unter dem Einfluss des enthaltenen Nachschäummittels den eigentlichen Schaum, beispielsweise einen Rasierschaum. Erfindungsgemäß vorteilhafte Zubereitungen liegen oft in speziellen Ausführungsformen wie etwa nachschäumenden Rasiergelen oder dergleichen vor.

Ferner vorteilhaft im Sinne der vorliegenden Erfindung sind After-shave Formulierungen (Formulierungen, welche nach einer Rasur des Gesichts oder anderer behaarter Körperteile (wie beispielsweise der Beine, der Achsel oder des Intimbereichs) angewendet werden) auf Basis erfindungsgemäßer Fettsäuren. Der pH-Wert solcher Zubereitungen liegt vorzugsweise im Bereich von 6 bis 8.

Ferner ist es auch möglich und gegebenenfalls vorteilhaft, die erfindungsgemäßen 4-Methyl-4-aryl-2-pentanole mit anderen Wirkstoffen zu kombinieren, beispielsweise mit anderen antimikrobiell, antimycotisch bzw. antiviral wirksamen Stoffen.

Besonders vorteilhafte Zubereitungen im Sinne der vorliegenden Erfindung enthalten neben den erfindungsgemäßen Wirkstoffen ferner eine oder mehrere verzweigte Fettsäuren, welche sich durch die folgende Strukturformel auszeichnen: 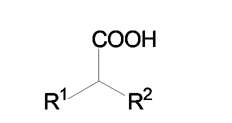 wobei R¹ einen verzweigten oder unverzweigten Alkylrest mit 1 bis 12 Kohlenstoffatomen und R² einen verzweigten oder unverzweigten Alkylrest mit 1 bis 24 Kohlenstoffatomen darstellen.

Vorteilhaft wird R¹ aus der Gruppe Methyl, Ethyl, Propyl, Butyl, Pentyl oder Hexyl gewählt. Weiter vorteilhaft wird R² aus der Gruppe Octyl, Nonyl, Decyl, Undecyl, Dodecyl gewählt.

Erfindungsgemäß besonders vorteilhaft ist es, die verzweigten Fettsäuren (eine oder mehrere Verbindungen) aus der Gruppe 2-Butyloctansäure, 2-Butyldecansäure, 2-Hexyloctansäure, 2-Hexyldecansäure zu wählen.

Ganz besonders bevorzugt sind die 2-Butyloctansäure und/oder 2-Hexyldecansäure.

Durch den Einsatz einer oder mehrerer verzweigter Fettsäuren wird die Wirksamkeit der erfindungsgemäßen Wirkstoffe in synergistischer Weise verbessert. Darüber hinaus zeigen erfindungsgemäße Zubereitungen, welche verzweigte Fettsäuren enthalten, eine verzögerte Freisetzung der 4-Methyl-4-aryl-2-pentanole. D. h. mit Hilfe von verzweigten Fettsäuren lässt sich eine längere, gleichmäßigere Wirksamkeit der erfindungsgemäßen Zubereitungen erreichen und ihre Wirkdauer steuern.

Gegenstand der Erfindung sind daher ferner auch die
Verwendung von verzweigten Fettsäuren, welche sich durch die folgende Strukturformel auszeichnen: 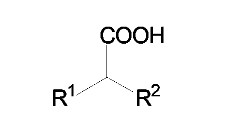 wobei R¹ einen verzweigten oder unverzweigten Alkylrest mit 1 bis 12 Kohlenstoffatomen und R² einen verzweigten oder unverzweigten Alkylrest mit 1 bis 24 Kohlenstoffatomen darstellen, zur Verstärkung der Wirksamkeit von 4-Methyl-4-aryl-2-pentanolen in kosmetischen oder dermatologischen Zubereitungen
und die
Verwendung von verzweigten Fettsäuren, welche sich durch die folgende Strukturformel auszeichnen: 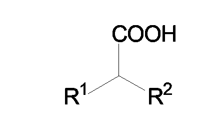 wobei R¹ einen verzweigten oder unverzweigten Alkylrest mit 1 bis 12 Kohlenstoffatomen und R² einen verzweigten oder unverzweigten Alkylrest mit 1 bis 24 Kohlenstoffatomen darstellen zur Steuerung der Wirkzeit von 4-Methyl-4-aryl-2-pentanolen in kosmetischen oder dermatologischen Zubereitungen.

Erfindungsgemäß bevorzugt werden die verzweigten Fettsäuren (eine oder mehrere Verbindungen) in einem Gehalt von 0,005 bis 10 Gew.-%, insbesondere 0,01 bis 5 Gew.-%, ganz besonders vorteilhaft 0,1 bis 3,0 Gew.-%, jeweils bezogen auf das Gesamtgewicht der Zubereitung, eingesetzt.

Die erfindungsgemäßen kosmetischen Zubereitungen können ferner kosmetische Hilfsstoffe enthalten, wie sie üblicherweise in solchen Zubereitungen verwendet werden, z. B. Konservierungsmittel, Lichtschutzfilter, Bakterizide, Antioxidantien, Parfüme, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Ebenso wie Emulsionen von flüssiger und fester Konsistenz als kosmetische Reinigungslotionen bzw. Reinigungscremes Verwendung finden, können auch die erfindungsgemäßen Zubereitungen Reinigungszubereitungen darstellen, welche beispielsweise zum Entfernen von Schminken und/oder Make-up oder als milder Waschschaum - ggf. auch für unreine Haut - verwendet werden können. Derartige Reinigungszubereitungen können vorteilhaft ferner als sogenannte "rinse off" Präparate angewendet werden, welche nach der Anwendung von der Haut abgespült werden.

Erfindungsgemäße Zubereitungen, die eine Reinigungszubereitung, also z. B. ein Shampoonierungsmittel, eine Wasch-, Dusch- oder Badezubereitung oder eine Seife darstellen, enthalten vorzugsweise mindestens eine anionische, nicht-ionische oder amphotere oberflächenaktive Substanz oder Gemische daraus und Hilfsmittel, wie sie üblicherweise dafür verwendet werden.

Beispiele für oberflächenaktive Substanzen, die erfindungsgemäß vorteilhaft verwendet werden können, sind herkömmliche Seifen, z. B. Fettsäuresalze des Natriums, Alkylsulfate, Alkylethersulfate, Alkan- und Alkylbenzolsulfonate, Sulfoacetate, Sulfobetaine, Sarcosinate, Amidosulfobetaine, Sulfosuccinate, Sulfobernsteinsäurehalbester, Alkylethercarboxylate, Eiweiß-Fettsäure-Kondensate, Alkylbetaine und Amidobetaine, Fettsäurealkanolamide, Polyglycolether-Derivate.

Die oberflächenaktive Substanz kann in einer Konzentration zwischen 1 und 50 Gew.-% in der Reinigungszubereitung (bezogen auf das Gesamtgewicht der Zubereitung) vorliegen.

Die erfindungsgemäßen kosmetischen und/oder dermatologischen Zubereitungen können auch vorteilhaft in Form eines Produktes zur Pflege des Haars bzw. der Kopfhaut (beispielsweise eines Antischuppen-Produkts) vorliegen, insbesondere eines Schaums zum Einlegen der Haare, eines Schaums, der beim Fönen der Haare verwendet wird, eines Frisier- und Behandlungsschaums. Auch Nagelpflegeprodukte sind bevorzugt im Sinne der vorliegenden Erfindung.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Die kosmetischen oder dermatologischen Mittel gemäß der Erfindung können beispielsweise aus Aerosolbehältern entnommen und dabei aufgeschäumt werden. Erfindungsgemäße Aerosolbehälter sind Sprühvorrichtungen mit einer Füllung aus den flüssigen bzw. breiartigen Stoffen, die unter dem Druck eines Treibmittels stehen (Druckgas- oder Aerosolpackungen). Deratige Behälter können mit Ventilen sehr unterschiedlicher Bauart ausgestattet sein, die die Entnahme des Inhalts als Schaum ermöglichen.

Als Treibmittel sind die üblichen "klassischen" leichtflüchtigen, verflüssigten Treibgase, wie beispielsweise Dimethylether (DME) und/oder lineare oder verzweigtkettige Kohlenwasserstoffe mit zwei bis fünf Kohlenstoffatomen (wie insbesondere Ethan, Propan, Butan, Isobutan und/oder Pentan) geeignet, die allein oder in Mischung miteinander eingesetzt werden können.

Auch Druckluft sowie weitere unter Druck befindliche Gase wie Luft, Sauerstoff, Stickstoff, Wasserstoff, Helium, Krypton, Xenon, Radon, Argon, Lachgas (N₂O) und Kohlendioxid (CO₂) sind vorteilhaft im Sinne der vorliegenden Erfindung als Treibgase (sowohl einzeln als in beliebigen Mischungen miteinander) zu verwenden.

Natürlich weiß der Fachmann, daß es weitere an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere halogenierte (mit Fluor, Clor, Brom, lod und/oder Astat substituierte) Kohlenwasserstoffe wie beispielsweise Fluorkohlenwasserstoffe und Fluorchlorkohlenwasserstoffe (FCKW).

Die genannten Gase können im Sinne der vorliegenden Erfindung jeweils einzeln oder in beliebigen Mischungen zueinander verwendet werden.

Vorteilhaft im Sinne der vorliegenden Erfindung wird der Volumenanteil an Treibgas aus dem Bereich von 0,1 bis 30 Vol.-%, bezogen auf das Gesamtvolumen aus Füllgut und Treibgas gewählt (entsprechend einem Volumenanteil von 70 bis 99,9 Vol.-% Füllgut).

Sollen die Zubereitungen im Sinne der vorliegenden Erfindung konserviert werden, so sind beispielsweise Formaldehydabspalter (wie z. B. DMDM Hydantoin), lodopropylbutylcarbamate (z. B. die unter den Handelsbezeichnungen Koncyl-L, Koncyl-S und Konkaben LMB von der Fa. Lonza erhältlichen), Parabene, Phenoxyethanol, Ethanol, Benzoesäure und dergleichen mehr vorteilhafte Konservierungsmittel. Üblicherweise umfaßt das Konservierungssystem erfindungsgemäß ferner vorteilhaft auch Konservierungshelfer, wie beispielsweise Octoxyglycerin, Glycine Soja etc.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber den noch fakultativ zu verwendende Antioxidantien können alle für kosmetische und/oder dermatologische Anwendungen geeigneten oder gebräuchlichen Antioxidantien verwendet werden.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, Polymere, Schaumstabilisatoren, Elektrolyte sowie Moisturizer.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

### Aerosol-Rasierschaum:

| | **I** | **II** | **III** |
|---|---|---|---|
| Stearinsäure | 6,00 | 6,00 | 6,00 |
| Myristinsäure | 1,00 | 1,00 | 1,00 |
| Polyethylenglykol(20)cetylether | 2,00 | 2,00 | 2,00 |
| Glycerin | 5,00 | 5,00 | 5,00 |
| Triethanolamin | 4,00 | 4,00 | 4,00 |
| 4-Methyl-4-aryl-2-pentanol | 0,20 | 0,30 | 0,30 |
| 2-Butyloctansäure | - | 0,10 | |
| 2-Hexyldecansäure | 0,20 | - | - |
| Parfum, Konservierungsstoffe | q.s. | q.s. | q.s. |
| Wasser | ad 100,00 | ad 100,00 | ad 100,00 |

Die durch Zusammenmischung der jeweiligen Bestandteile erhaltene flüssige Phase wird mit einem Propan-Isobutan-Gemisch (2:7) im Verhältnis 3:97 in Aerosolbehälter abgefüllt.

### Aerosol-Rasiergel:

| | **I** | **II** | **III** |
|---|---|---|---|
| Palmitininsäure | 7,00 | 7,00 | 7,00 |
| Stearinsäure | 1,50 | 1,50 | 1,50 |
| Polyethylenglykol(25)cetearylether | 2,00 | 2,00 | 2,00 |
| Sorbitol | 5,50 | 5,50 | 5,50 |
| Triethanolamin | 5,00 | 5,00 | 5,00 |
| Hydroxyethylcellulose | 0,30 | 0,30 | 0,30 |
| 4-Methyl-4-aryl-2-pentanol | 0,20 | 0,30 | 0,30 |
| 2-Butyloctansäure | - | 0,10 | - |
| 2-Hexyldecansäure | 0,20 | - | - |
| Parfum, Konservierungsstoffe | q.s. | q.s. | q.s. |
| Wasser | ad 100,00 | ad 100,00 | ad 100,00 |

Die durch Zusammenmischung der jeweiligen Bestandteile erhaltene flüssige Phase wird mit einem n-Pentan-n-Butan-Gemisch (5:1) im Verhältnis 6:94 in Aerosolbehälte r abgefüllt.

### Deo-Stift Typ A

| | **I** | **II** | **III** |
|---|---|---|---|
| Natriumstearat | 7,00 | 7,00 | 7,00 |
| 1,2-Propylenglycol | 48,00 | 48,00 | 48,00 |
| 4-Methyl-4-aryl-2-pentanol | 0,20 | 0,30 | 0,30 |
| 2-Butyloctansäure | - | 0,10 | |
| 2-Hexyldecansäure | 0,20 | - | - |
| Parfum, Konservierungsstoffe | q.s. | q.s. | q.s. |
| Wasser | ad 100,00 | ad 100,00 | ad 100,00 |

### Deo-Stift Typ B

| | **I** | **II** | **III** |
|---|---|---|---|
| Natriumstearat | 8,00 | 8,00 | 8,00 |
| 1,2-Propylenglycol | 45,00 | 45,00 | 45,00 |
| 4-Methyl-4-aryl-2-pentanol | 0,20 | 0,30 | 0,30 |
| 2-Butyloctansäure | - | 0,50 | - |
| 2-Hexyldecansäure | 0,50 | - | - |
| Polyethylenglycol(25)cetearylether | 3,00 | 3,00 | 3,00 |
| Ethanol | 20,00 | 20,00 | 20,00 |
| Parfum, Konservierungsstoffe | q.s. | q.s. | q.s. |
| Wasser | ad 100,00 | ad 100,00 | ad 100,00 |

### After shave balsam

| | **I** | **II** | **III** |
|---|---|---|---|
| Natriumstearat | 2,5 | 2,5 | 2,5 |
| Glycerin | 10,00 | 10,00 | 10,00 |
| 4-Methyl-4-aryl-2-pentanol | 0,20 | 0,30 | 0,30 |
| 2-Butyloctansäure | - | 0,50 | - |
| 2-Hexyldecansäure | 0,50 | - | - |
| Cetyl Alkohol | 3,00 | 3,00 | 3,00 |
| Diemethicone | 2,00 | 2,00 | 2,00 |
| Dicaprylyl Carbonat | 2,00 | 2,00 | 2,00 |
| Parfum, Konservierungsstoffe | q.s. | q.s. | q.s. |
| Wasser | ad 100,00 | ad 100,00 | ad 100,00 |

## Patentansprüche

1. Kosmetische oder dermatologische Zubereitungen enthaltend ein oder mehrere 4-Methyl-4-aryl-2-pentanole, **dadurch gekennzeichnet, dass** sie eine oder mehrere mit organischen oder anorganischen Basen neutralisierte, geradkettige Fettsäuren mit 14 bis 18 Kohlenstoffatomen enthalten, wobei der pH-Wert der Zubereitungen zwischen 6 und 11 liegt.

2. Zubereitung nach Anspruch 1, **dadurch gekennzeichnet, daß** als 4-Methyl-4-aryl-2-pentanol das 4-Methyl-4-phenyl-2-pentanol gewählt wird.

3. Zubereitung nach einem der vorhergehenden Ansprüche, **dadurch gekennzeichnet, daß** als geradkettige Fettsäure Stearin-, Palmitin- und/oder Myristinsäure gewählt wird.

4. Zubereitung nach einem der vorhergehenden Ansprüche, **dadurch gekennzeichnet, daß** die geradkettige Fettsäure mit anorganischen Basen neutralisiert wird.

5. Zubereitung nach einem der vorhergehenden Ansprüche, **dadurch gekennzeichnet, daß** die geradkettige Fettsäure mit organischen Basen neutralisiert wird.

6. Zubereitung nach Anspruch 5, **dadurch gekennzeichnet, daß** als organische Base Triethanolamin gewählt wird.

7. Zubereitung nach einem der vorhergehenden Ansprüche, **dadurch gekennzeichnet, daß** sie ferner eine oder mehrere verzweigte Fettsäuren enthält, welche sich durch die folgende Strukturformel auszeichnen: 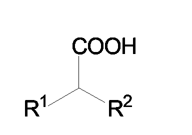 wobei R¹ einen verzweigten oder unverzweigten Alkylrest mit 1 bis 12 Kohlenstoffatomen und R² einen verzweigten oder unverzweigten Alkylrest mit 1 bis 24 Kohlenstoffatomen darstellen.

8. Zubereitung nach einem der vorhergehenden Ansprüche, **dadurch gekennzeichnet, daß** sie in Form eines Stiftes vorliegt.

9. Zubereitung nach einem der Ansprüche 1 bis 7, **dadurch gekennzeichnet, daß** sie in Form eines schaumförmige oder nachschäumende Aerosols vorliegt.

10. Zubereitung nach einem der Ansprüche 1 bis 7, **dadurch gekennzeichnet, daß** sie in Form einer After shave-Formulierung, welche nach einer Rasur angewendet wird, vorliegt.

11. Verwendung von kosmetischen oder dermatologischen Zubereitungen enthaltend mit organischen oder anorganischen Basen neutralisierte, geradkettige Fettsäuren mit 14 bis 18 Kohlenstoffatomen zur Verhinderung oder Verzögerung der Penetration von 4-Methyl-4-aryl-2-pentanolen in die Haut.

12. Verwendung von verzweigten Fettsäuren, welche sich durch die folgende Strukturformel auszeichnen: 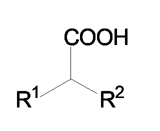 wobei R¹ einen verzweigten oder unverzweigten Alkylrest mit 1 bis 12 Kohlenstoffatomen und R² einen verzweigten oder unverzweigten Alkylrest mit 1 bis 24 Kohlenstoffatomen darstellen, zur Verstärkung der Wirksamkeit von 4-Methyl-4-aryl-2-pentanolen in kosmetischen oder dermatologischen Zubereitungen.

13. Verwendung von verzweigten Fettsäuren, welche sich durch die folgende Strukturformel auszeichnen: 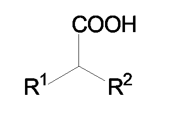 wobei R¹ einen verzweigten oder unverzweigten Alkylrest mit 1 bis 12 Kohlenstoffatomen und R² einen verzweigten oder unverzweigten Alkylrest mit 1 bis 24 Kohlenstoffatomen darstellen, zur Steuerung der Wirkzeit von 4-Methyl-4-aryl-2-pentanolen in kosmetischen oder dermatologischen Zubereitungen.
